(19) Europäisches Patentamt
European Patent Office
Office européen des brevets

(11) Veröffentlichungsnummer: **0 111 205**
**B1**

(12) # EUROPÄISCHE PATENTSCHRIFT

(45) Veröffentlichungstag der Patentschrift: **01.04.87**

(21) Anmeldenummer: **83111672.8**

(22) Anmeldetag: **22.11.83**

(51) Int. Cl.⁴: **C 07 D 417/12,** A 61 K 31/435 // C07D219/06, C07D219/10, (C07D417/12, 277:00, 219:10)

(54) **Acridanon-Derivate.**

(30) Priorität: **26.11.82 CH 6895/82**

(43) Veröffentlichungstag der Anmeldung: **20.06.84 Patentblatt 84/25**

(45) Bekanntmachung des Hinweises auf die Patenterteilung: **01.04.87 Patentblatt 87/14**

(84) Benannte Vertragsstaaten: **AT BE CH DE FR GB IT LI LU NL SE**

(56) Entgegenhaltungen:
**DE - A - 1 811 409**
**GB - A - 1 068 595**

(73) Patentinhaber: **F. HOFFMANN-LA ROCHE & CO. Aktiengesellschaft, CH-4002 Basel (CH)**

(72) Erfinder: **Bretschneider, Hermann, Dr., Nikodemweg 11, Innsbruck (AT)**
Erfinder: **Brombacher, Urs, Im Hirshalm 35, Riehen (CH)**
Erfinder: **Montavon, Marc, Dr., Realpstrasse 72, Basel (CH)**
Erfinder: **Schantl, Joachim, Prof. Dr., Karl-Innerebner-Strasse 84, Innsbruck (AT)**
Erfinder: **Türk, Dr. Wolfgang, Prankhstrasse 28, Ludwigshafen am Rhein (DE)**

(74) Vertreter: **Lederer, Franz, Dr. et al, Vanderwerth, Lederer & Riederer Patentanwälte Lucile-Grahn-Strasse 22, D-8000 München 80 (DE)**

EP 0 111 205 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Acridanon-Derivate der allgemeinen Formel

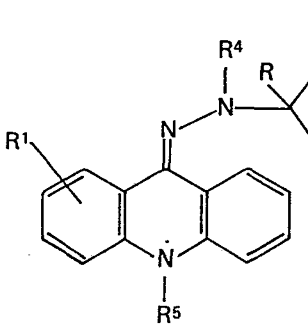

I

worin die punktierte Linie eine fakultative Bindung, $R^1$ Wasserstoff, Halogen oder Nitro, $R^2$ Wasserstoff oder niederes Alkyl, einer der Substituenten $R^3$ und $R^4$ Wasserstoff oder niederes Alkyl und der andere zusammen mit R eine zusätzliche Bindung und $R^5$ Wasserstoff, niederes Alkyl, niederes Alkenyl, niederes Alkinyl oder durch Halogen oder niederes Alkoxy substituiertes niederes Alkyl bedeuten, und pharmazeutisch annehmbare Säureadditionssalze davon.

Diese neuen Stoffe besitzen wertvolle pharmakologische Eigenschaften und können bei der Bekämpfung oder Verhütung von Krankheiten verwendet werden.

Gegenstand der vorliegenden Erfindung sind Acridanon-Derivate der obigen Formel I und ihre pharmazeutisch annehmbaren Salze als solche und als pharmazeutische Wirkstoffe, die Herstellung dieser Stoffe und Zwischenprodukte zu deren Herstellung, sowie diese enthaltende Arzneimittel und die Herstellung solcher Arzneimittel.

Der Ausdruck «nieder», wie er in der vorliegenden Beschreibung verwendet wird, bedeutet, dass die derart bezeichneten Verbindungen oder Reste bis zu 4 Kohlenstoffatome enthalten und geradkettig oder verzweigt sein können. Der Ausdruck «niederes Alkyl» bezeichnet gesättigte Kohlenwasserstoffreste, wie Methyl, Äthyl, n-Butyl und dergleichen. Der Ausdruck «niederes Alkenyl» bezeichnet Kohlenwasserstoffreste, welche eine olefinische Doppelbindung enthalten, wie Allyl und dergleichen. Der Ausdruck «niederes Alkinyl» bezeichnet Kohlenwasserstoffreste mit einer Dreifachbindung, wie Propargyl und dergleichen. Der Ausdruck «niederes Alkoxy» bezeichnet über ein Sauerstoffatom gebundene niedere Alkylgruppen, wie Methoxy und dergleichen. Der Ausdruck «durch Halogen oder niederes Alkoxy substituiertes niederes Alkyl» umfasst Reste, wie 2-Chloräthyl, 2-Methoxyäthyl, 2,2-Dimethoxyäthyl und dergleichen. Der Ausdruck «Halogen» bedeutet Fluor, Chlor, Brom oder Jod.

Die Verbindungen der obigen Formel I können, je nach Bedeutung der punktierten Linie und der Substituenten R, $R^3$, $R^4$ und $R^5$ in verschiedenen automeren Formen vorliegen; die vorliegende Erfindung umfasst sämtliche möglichen automeren Formen.

Die bevorzugte Bedeutungsmöglichkeit von $R^1$ ist Wasserstoff. $R^2$ bedeutet vorzugsweise Wasserstoff. Vorzugsweise bedeutet einer der Substituenten $R^3$ und $R^4$ Wasserstoff und der andere zusammen mit R eine zusätzliche Bindung. $R^5$ bedeutet vorzugsweise Wasserstoff oder niederes Alkyl.

Eine im Rahmen der vorliegenden Erfindung ganz besonders bevorzugte Verbindung ist 10-Methyl-9-acridanon-(2-thiazolyl)hydrazon.

Weitere bevorzugte Verbindungen der Formel I sind: 9-Acridanon-(2-thiazolidinyliden)hydrazon, 9-Acridanon-(2-thiazolyl)hydrazon, 10-Methyl-9-acridanon-(2-thiazolidinyliden)hydrazon, 10-Äthyl-9-acridanon-(2-thiazolidinyliden)hydrazon, 9-Acridanon-methyl(2-thiazolinyl)hydrazon und 10-(2-Methoxyäthyl)-9-acridanon-(2-thiazolidinyliden)hydrazon.

Die Acridanon-Derivate der eingangs definierten Formel I und ihre pharmazeutisch annehmbaren Säureadditionssalze können erfindungsgemäss dadurch hergestellt werden, dass man

a) eine Verbindung der allgemeinen Formel

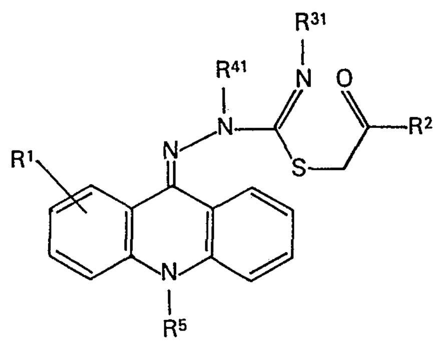

II

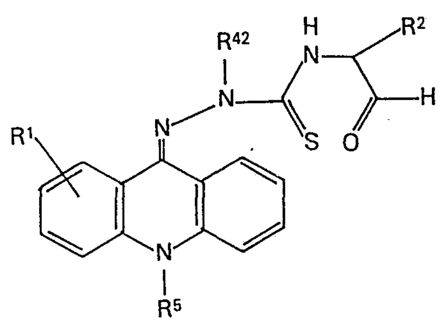

III

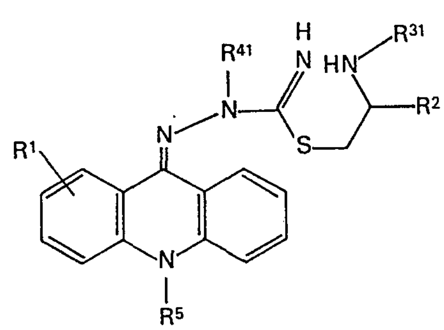

IV

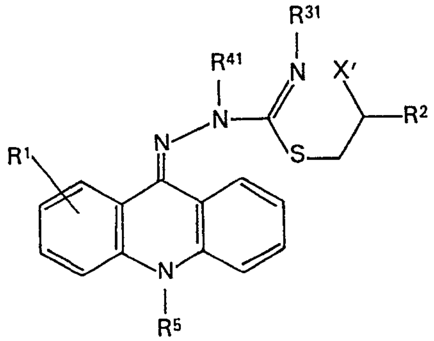

V

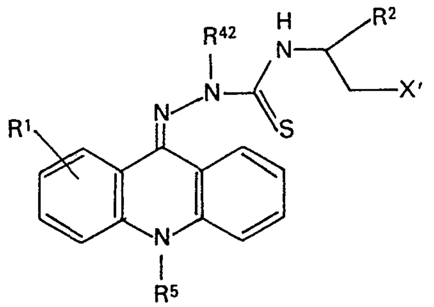

VI

oder

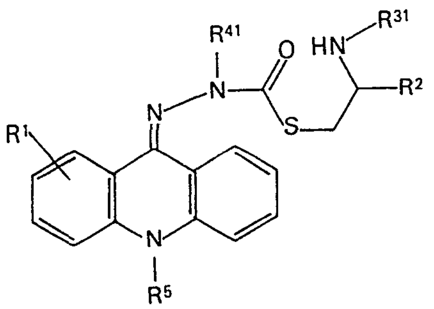

VII

worin einer der Substituenten $R^{31}$ und $R^{41}$ Wasserstoff oder niederes Alkyl und der andere Wasserstoff, $R^{42}$ Wasserstoff oder niederes Alkyl und X′ eine Abgangsgruppe bedeuten, und $R^1$, $R^2$ und $R^5$ obige Bedeutung besitzen, cyclisiert, oder

b) eine Verbindung der allgemeinen Formel

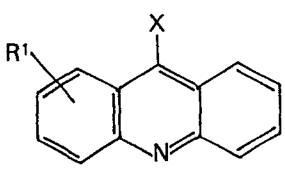

VIII

oder

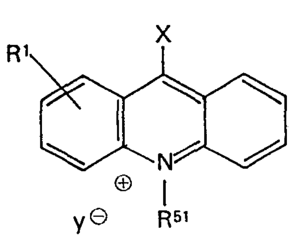

IX

worin $R^{51}$ niederes Alkyl, niederes Alkenyl, niederes Alkinyl oder durch Halogen oder niederes Alkoxy substituiertes niederes Alkyl, X eine Abgangsgruppe und $Y^{\ominus}$ ein Anion bedeuten, und $R^1$ obige Bedeutung besitzt,

mit einer Verbindung der allgemeinen Formel

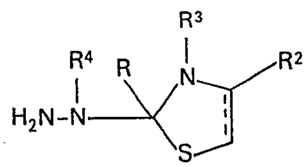

X

worin die punktierte Linie, R, $R^2$, $R^3$ und $R^4$ obige Bedeutung besitzen, umsetzt, oder

c) eine Verbindung der allgemeinen Formel

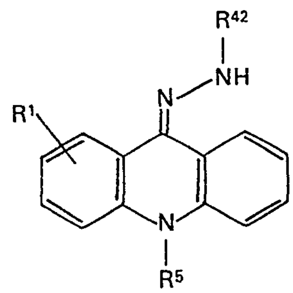

XI

worin $R^{42}$ Wasserstoff oder niederes Alkyl bedeutet, und $R^1$ und $R^5$ obige Bedeutung besitzen, mit einer Verbindung der allgemeinen Formel

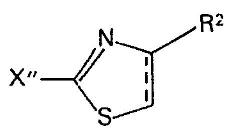

XII

worin X″ eine Abgangsgruppe bedeutet, und $R^2$ und die punktierte Linie obige Bedeutung besitzen, umsetzt, und

d) erwünschtenfalls eine erhaltene Verbindung der Formel I in ein pharmazeutisch annehmbares Säureadditionssalz überführt.

Gemäss Verfahrensvariante a) können die Verbindungen der Formel I hergestellt werden, indem man eine Verbindung der Formel II, III, IV, V, VI oder VII nach an sich bekannten und jedem Fachmann geläufigen Methoden cyclisiert.

Die in den Formeln V und VI mit X′ bezeichnete Abgangsgruppe ist vorzugsweise ein Halogenatom, insbesondere ein Brom- oder Chloratom. Die Ringschlussreaktion erfolgt je nach verwendetem Ausgangsstoff ziemlich leicht und kann gegebenenfalls durch längeres Stehenlassen und/oder Anwendung von Wärme bewerkstelligt oder vervollständigt werden. Die für die Ringschlussreaktion verwendeten Ausgangsstoffe müssen nicht notwendigerweise in isoliertem Zustand eingesetzt werden; in der Regel erweist es sich als zweckmässig, diese Ausgangsstoffe ohne Isolierung aus dem Reaktionsgemisch, in dem sie hergestellt worden sind, direkt zu cyclisieren bzw. cyclisieren zu lassen. Je nach angewendeten Reaktionsbedingungen ist in einigen

Fällen eine Isolierung gar nicht möglich, da die Cyclisierung spontan erfolgt.

Für den vorliegenden Verfahrensaspekt geeignete Lösungsmittel sind beispielsweise Äther, wie Tetrahydrofuran, Dioxan, Äthylenglykol-dimethyläther, Diäthylenglykol-dimethyläther und dergleichen, Alkohole, wie Methanol, Äthanol und dergleichen, Dimethylformamid, Dimethylsulfoxid, Acetonitril und dergleichen. Die Reaktionstemperatur liegt zweckmässigerweise in einem Bereich von etwa Raumtemperatur bis zur Siedetemperatur der Reaktionsmischung.

Gemäss Verfahrensvariante b) können die Verbindungen der Formel I dadurch hergestellt werden, dass man eine Verbindung der Formel VIII oder IX mit einer Verbindung der Formel X umsetzt. Die in den Verbindungen der Formeln VIII und IX mit X bezeichnete Abgangsgruppe ist vorzugsweise ein Halogenatom, eine niedere Alkanoyloxygruppe oder eine niedere Alkoxygruppe, insbesondere ein Chloratom, eine Acetyloxygruppe oder eine Methoxygruppe. Währenddem die Verbindungen der Formel VIII in der Regel stabile Substanzen sind, ist dies bei den Verbindungen der Formel IX nicht immer der Fall. Die Verbindungen der Formel IX werden deshalb zweckmässigerweise kurz vor der Reaktion mit einer Verbindung der Formel X aus einer Verbindung der allgemeinen Formel

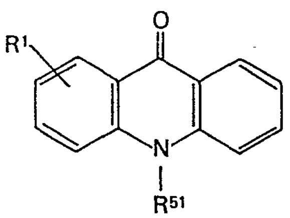

XIII

worin $R^1$ und $R^{51}$ obige Bedeutung besitzen,

wie weiter unten beschrieben hergestellt und, gegebenenfalls ohne vorgängige Isolierung, direkt weiterverarbeitet.

Die Verbindung der Formel X wird zweckmässigerweise in Form eines Säureadditionssalzes eingesetzt, beispielsweise in Form eines Hydrochlorides oder Hydrobromides. Man kann die Reaktion in Gegenwart eines säurebindenden Mittels durchführen, wobei als säurebindende Mittel insbesondere Natrium- und Kaliumcarbonate, -bicarbonate und -acetate in Frage kommen. Als Lösungsmittel für den vorliegenden Verfahrensaspekt eignen sich beispielsweise niedere Alkohole, wie Methanol und Äthanol, und andere unter den Reaktionsbedingungen inerte organische Lösungsmittel, wie Dimethylformamid, Acetonitril und dergleichen. Die Reaktionstemperatur liegt zweckmässigerweise in einem Bereich von etwa Raumtemperatur bis zur Siedetemperatur der Reaktionsmischung.

Gemäss Verfahrensvariante c) können die Verbindungen der Formel I durch Umsetzen einer Verbindung der Formel XI mit einer Verbindung der Formel XII erhalten werden. Die in der Formel XII mit X″ bezeichneten Abgangsgruppe ist vorzugsweise ein Halogenatom, z.B. ein Chlor- oder Bromatom, oder die Thiolgruppe. Die folgenden, unter den Reaktionsbedingungen inerten organischen Lösungsmittel können verwendet werden: Äther, wie Tetrahydrofuran, Dioxan, Diäthyläther und dergleichen, Alkohole, wie Methanol, Äthanol und dergleichen, Dimethylformamid, Dimethylsulfoxid, Acetonitril und dergleichen. Die Reaktionstemperatur liegt zweckmässigerweise in einem Bereich von etwa Raumtemperatur bis zur Siedetemperatur der Reaktionsmischung.

Gemäss Verfahrensvariante d) können die Acridanon-Derivate der eingangs definierten Formel I in pharmazeutisch annehmbare Säureadditionssalze übergeführt werden. Die Herstellung von solchen Säureadditionssalzen erfolgt dabei nach allgemein üblichen Methoden. Es kommen sowohl Salze mit pharmazeutisch annehmbaren anorganischen als auch Salze mit pharmazeutisch annehmbaren organischen Säuren in Betracht, beispielsweise Hydrochloride, Hydrobromide, Sulphate, Citrate, Acetate, Succinate, Methansulfonate, p-Toluolsulfonate und dergleichen.

Die als Ausgangsstoffe verwendeten Verbindungen der Formeln II, III, IV, V, VI, VII und XI, worin $R^5$ Wasserstoff bedeutet, können ausgehend von Verbindungen der Formel VIII gemäss dem nachfolgenden Formelschema I hergestellt werden; die Substituenten $R^1$, $R^2$, $R^{31}$, $R^{41}$, $R^{42}$, X und X′ besitzen obige Bedeutung, $R^6$ und $R^7$ bedeuten je niederes Alkyl oder zusammen niederes Alkylen, und $R^8$ bedeutet niederes Alkyl, Phenyl oder substituiertes Phenyl.

Die als Ausgangsstoffe verwendeten Verbindungen der Formel VIII sind bekannt oder können in Analogie zu den bekannten Vertretern dieser Stoffklasse hergestellt werden.

Formelschema I

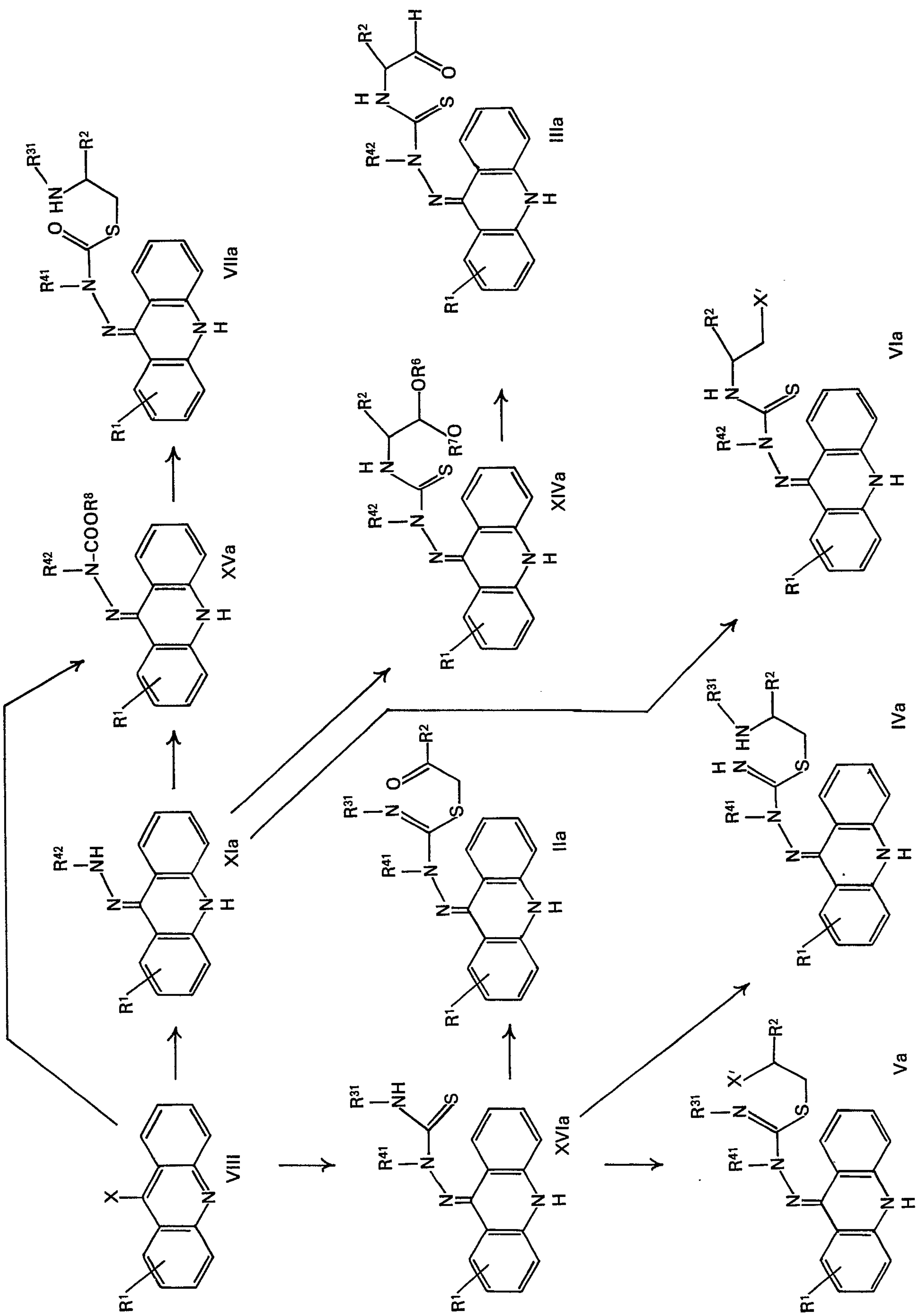

VIII XIa XVa VIIa

XVIa IIa XIVa IIIa

Va IVa VIa

Die Verbindungen der allgemeinen Formeln XIa und XVIa können durch Umsetzen einer Verbindung der Formel VIII mit einem Hydrazin der allgemeinen Formel $H_2N-NH-R^{42}$, worin $R^{42}$ obige Bedeutung besitzt, bzw. mit einem Thiosemicarbazid der allgemeinen Formel $H_2N-NR^{41}-CS-NHR^{31}$, worin $R^{31}$ und $R^{41}$ obige Bedeutung besitzen, hergestellt werden, wobei man die weiter oben für die Verfahrensvariante b), angegebenen Reaktionsbedingungen anwenden kann.

Durch Umsetzen einer Verbindung der Formel XVIa mit einer Verbindung der allgemeinen Formel $X'-CH_2COR^2$, worin X' und $R^2$ obige Bedeutung besitzen, erhält man eine Verbindung der Formel IIa. Die mit X' bezeichnete Abgangsgruppe ist vorzugsweise ein Chlor- oder Bromatom. Als Lösungsmittel eignen sich insbesondere niedere Alkohole, wie Methanol und Äthanol. Die Reaktionstemperatur liegt zweckmässigerweise in einem Bereich von etwa Raumtemperatur bis Siedetemperatur der Reaktionsmischung.

Verbindungen der Formel IVa können erhalten werden, indem man eine Verbindung der Formel XVIa, worin $R^{31}$ Wasserstoff bedeutet, mit einer Verbindung der allgemeinen Formel $X'-CH_2-CHR^2-NHR^{32}$, worin X' und $R^2$ obige Bedeutung besitzen, und $R^{32}$ Wasserstoff oder, falls $R^{41}$ in der Verbindung der Formel XVIa Wasserstoff bedeutet, auch niederes Alkyl bedeutet, umsetzt. Das Amin wird dabei zweckmässigerweise in Form eines Säureadditionssalzes eingesetzt, wobei insbesondere die Hydrochloride oder Hydrobromide in Frage kommen. Als Lösungsmittel eignen sich beispielsweise niedere Alkohole, wie Methanol und Äthanol, Äther, wie Tetrahydrofuran, Dioxan und Äthylenglycoldimethyläther, Dimethylformamid, Dimethylsulfoxid, Acetonitril und dergleichen. Die Reaktionstemperatur liegt vorzugsweise zwischen etwa Raumtemperatur und Siedetemperatur der Reaktionsmischung.

Verbindungen der Formel Va können erhalten werden, indem man eine Verbindung der Formel XVIa in einem inerten organischen Lösungsmittel und in einem Bereich von etwa Raumtemperatur bis Siedetemperatur der Reaktionsmischung mit einer Verbindung der allgemeinen Formel $X'-CH_2-CHR^2-X'$, worin X' und $R^2$ obige Bedeutung besitzen, umsetzt, wobei man vorzugsweise ein Dichlorid oder Dibromid verwendet. Geeignete Lösungsmittel sind beispielsweise Alkohole, wie Methanol, Äthanol und dergleichen, Äther, wie Tetrahydrofuran, Dioxan und dergleichen, Dimethylformamid, Dimethylsulfoxid und dergleichen.

Die Verbindungen der Formel IIIa können hergestellt werden, indem man eine Verbindung der Formel XIa mit einem Isothiocyanat der allgemeinen Formel

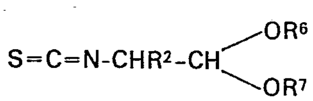 ,

worin $R^2$, $R^6$ und $R^7$ obige Bedeutung besitzen, umsetzt und anschliessend die Acetalgruppe in der erhaltenen Verbindung der allgemeinen Formel XIVa hydrolysiert. Der erste Schritt wird zweckmässigerweise in einem inerten organischen Lösungsmittel, z.B. in einem Äther, wie Diäthyläther, t-Butylmethyläther und Tetrahydrofuran, oder in Dimethylformamid, Acetonitril oder dergleichen, bei Temperaturen von etwa Raumtemperatur bis Siedetemperatur durchgeführt. Die Hydrolyse der Acetalgruppe kann mittels einer wässrigen Säure bewerkstelligt werden, wobei man gegebenenfalls in Gegenwart eines Lösungsvermittlers, wie Tetrahydrofuran, Dioxan, Methanol, Äthanol, Dimethylformamid oder dergleichen, arbeiten kann. Als Säuren kommen z.B. Schwefelsäure, Salzsäure, p-Toluolsulfonsäure und dergleichen in Betracht. Die Temperatur ist nicht kritisch und kann in einem weiten Bereich variieren.

Die Verbindungen der Formel VIa können hergestellt werden, indem man eine Verbindung der Formel XIa mit einem Isothiocyanat der allgemeinen Formel $S=C=N-CHR^2-CH_2-X'$, worin X' und $R^2$ obige Bedeutung besitzen, umsetzt. Man kann beispielsweise die weiter oben für die Herstellung von Verbindungen der Formel XIVa aus solchen der Formel XIa angegebenen Reaktionsbedingungen anwenden.

Die Verbindungen der Formel VIIa können hergestellt werden, indem man eine Verbindung der Formel XIa in an sich bekannter Weise mit einem den Rest $-COOR^8$ liefernden Mittel, z.B. einem Dialkyl- oder Diphenylcarbonat oder einem Chlorameisensäure-alkyl- oder phenylester, behandelt und die erhaltene Verbindung der allgemeinen Formel XVa mit einem Thiol der allgemeinen Formel $HS-CH_2-CH^2-NHR^{33}$, worin $R^2$ obige Bedeutung besitzt, und $R^{33}$ Wasserstoff oder, falls $R^{42}$ in der Verbindung der Formel XVa Wasserstoff bedeutet, auch niederes Alkyl bedeutet, umsetzt. Dieser zweite Schritt wird vorzugsweise zwischen etwa Raumtemperatur und Siedetemperatur der Reaktionsmischung in einem inerten organischen Lösungsmittel durchgeführt, wobei als Lösungsmittel insbesondere Äther, wie Diäthyläther, Tetrahydrofuran und dergleichen, und niedere Alkohole, wie Methanol und Äthanol, in Betracht kommen.

Die Verbindungen der Formel XVa können andererseits auch durch Umsetzen einer Verbindung der Formel VIII mit einem Hydrazin der allgemeinen Formel $H_2N-NR^{42}-COOR^8$, worin $R^{42}$ und $R^8$ obige Bedeutung besitzen, hergestellt werden, was in an sich bekannter Weise erfolgen kann, beispielsweise unter Anwendung der weiter oben für die Verfahrensvariante b) angegebenen Reaktionsbedingungen.

Wie bereits weiter oben erwähnt, ist es nicht notwendig (und in manchen Fällen auch nicht möglich), die Verbindungen der Formeln II, III, IV, V, VI und VII zu isolieren; vielmehr erweist es sich in der Regel als zweckmässig, diese Verbindungen ohne Isolierung an dem Reaktionsgemisch, in dem sie hergestellt worden sind, direkt zu cyclisieren bzw. cyclisieren zu lassen.

Die als Ausgangsstoffe verwendeten Verbindungen der Formel IX, sowie diejenigen der For-

meln II, III, IV, V, VI, VII und XI, worin $R^5$ niederes Alkyl, niederes Alkenyl, niederes Alkinyl oder durch Halogen oder niederes Alkoxy substituiertes niederes Alkyl bedeutet, können ausgehend von Verbindungen der Formel XIII gemäss dem nachfolgenden Formelschema II hergestellt werden; die Substituenten $R^1$, $R^2$, $R^{31}$, $R^{41}$, $R^{42}$, $R^{51}$, $R^6$, $R^7$, $R^8$, X und X′ besitzen obige Bedeutung. Die Verbindungen der Formel XIII gehören einer an sich bekannten Stoffklasse an.

Formelschema II

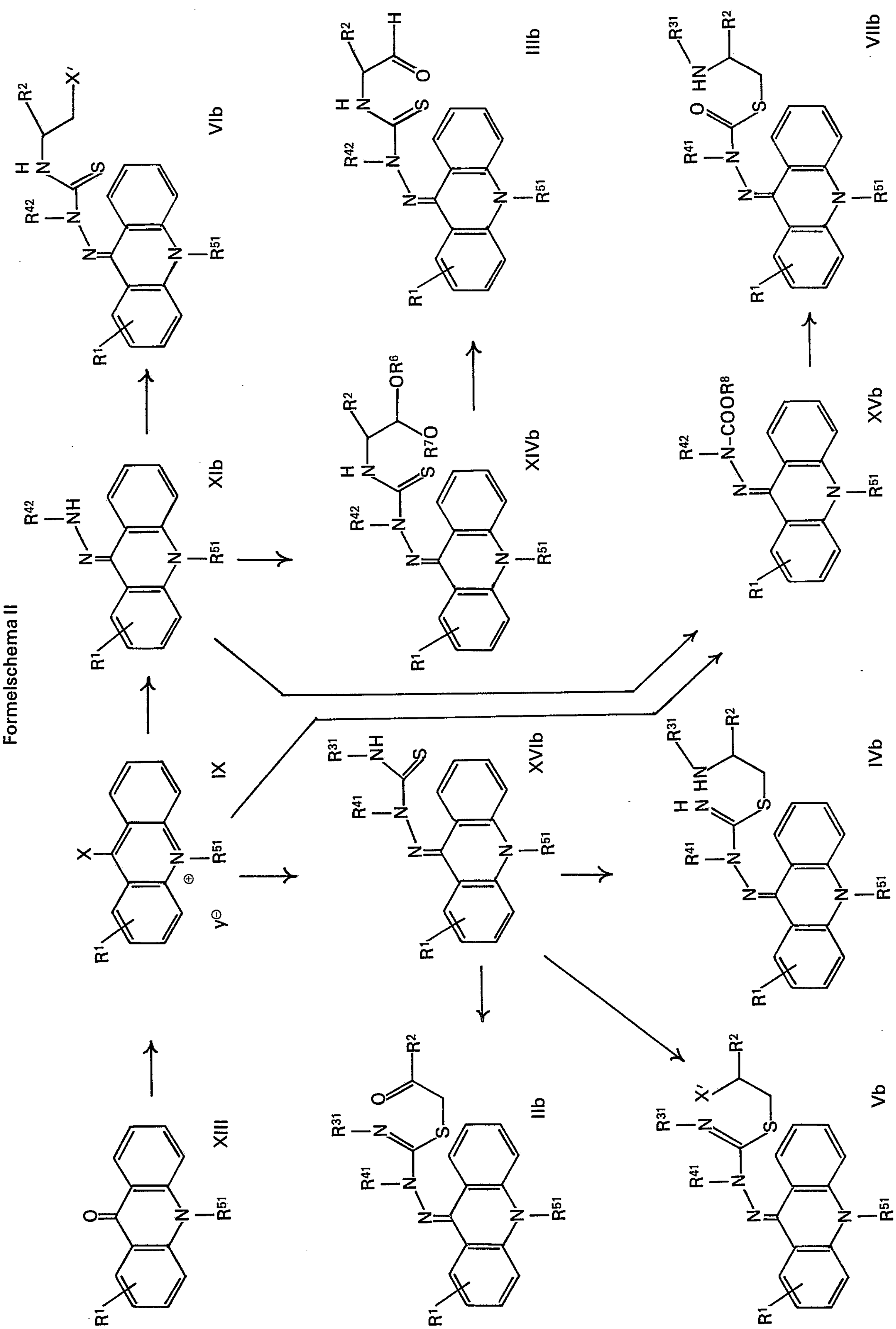

XIII IX XIb VIb IIb XVIb XIVb IIIb Vb IVb XVb VIIb

Diejenigen Verbindungen der Formel IX, worin X ein Halogenatom bedeutet, können hergestellt werden, indem man eine Verbindung der Formel XIII in einem inerten organischen Lösungsmittel mit einem Halogenierungsmittel behandelt. In einer bevorzugten Ausführungsform verwendet man als Halogenierungsmittel Oxalylchlorid oder Phosphoroxychlorid und als Lösungsmittel halogenierte Kohlenwasserstoffe, wie Methylenchlorid, Chloroform, 1,2-Dichloräthan oder dergleichen, Acetonitril oder überschüssiges Halogenierungsmittel, wobei man eine Verbindung der Formel IX erhält, worin X Chlor bedeutet. Die Reaktionstemperaturen variieren vorteilhafterweise in einem Bereich von etwa Raumtemperatur bis zur Siedetemperatur der Reaktionsmischung.

Verbindungen der Formel IX, worin X eine von Halogen verschiedene Abgangsgruppe bedeutet, können aus den entsprechenden Halogenverbindungen erhalten werden. Man kann beispielsweise das Halogenatom in einer solchen Verbindung in an sich bekannter Weise durch andere Abgangsgruppen, z.B. durch niedere Alkoxygruppen oder niedere Alkanoyloxygruppen, ersetzen.

Bei den Verbindungen der Formel IX handelt es sich um quarternäre Ammoniumsalze, wovon einige, wie bereits früher erwähnt, nicht besonders stabil sind; diese werden zweckmässigerweise unmittelbar nach ihrer Herstellung weiter verarbeitet. Die Natur des mit $Y^{\ominus}$ bezeichneten Anions hängt von der Art der Herstellung der entsprechenden Verbindung der Formel IX ab. Stellt man beispielsweise eine Verbindung der Formel IX her, worin X Chlor bedeutet, und verwendet als Halogenierungsmittel Oxalylchlorid, so erhält man eine Verbindung der Formel IX, worin $Y^{\ominus}$ ein Chloridanion ist; verwendet man als Halogenierungsmittel Phosphoroxychlorid so erhält man eine entsprechende Verbindung, worin $Y^{\ominus}$ $PO_2Cl_2{}^{\ominus}$ bedeutet.

Die Verbindungen der Formeln IIb, IIIb, IVb, Vb, VIb, VIIb und XIb können aus Verbindungen der Formel IX hergestellt werden, und zwar in Analogie zur Herstellung der entsprechenden Verbindungen, welche am Acridanon-Stickstoffatom durch Wasserstoff substituiert sind, aus Verbindungen der Formel VIII. Die als Ausgangsstoffe verwendeten Verbindungen der allgemeinen Formeln II, III, IV, V, VI und VII sind neu und ebenfalls Gegenstand der vorliegenden Erfindung.

Die Acridanon-Derivate der obigen Formel I und ihre pharmazeutisch annehmbaren Säureadditionssalze besitzen wertvolle pharmakologische Eigenschaften; sie entfalten insbesondere wertvolle schistosomizide Wirkungen und können demnach bei der Bekämpfung bzw. Verhütung von Schistosomiasen verwendet werden.

Die schistosomizide Wirkung der erfindungsgemässen Stoffe kann tierexperimentell wie folgt nachgewiesen werden:

Albino-Mäuse mit einem Gewicht von 15–18 g werden subcutan mit 60±5 Cercarien eines Liberia-Stammes von Schistosoma mansoni infiziert. 46 Tage nach der Infektion werden die Tiere einmal mit den zu prüfenden Substanzen peroral behandelt. Pro Substanz und Dosierung werden 5–10 Tiere eingesetzt. Als Kontrolle dienen 10 unbehandelte Tiere. Die Sektion erfolgte nach 60 Tagen, worauf Wurmpaare und einzelne Würmer in Mesenterialvenen, Pfortader und Leber herauspräpariert und gezählt werden. Die Präparat-Wirkung zeigt sich in einer reduzierten Zahl lebender Parasiten im Vergleich zu der Zahl in den Kontrolltieren.

Zur Auswertung wird die prozentuale Reduktion der Parasiten bei behandelten Tieren im Vergleich zu unbehandelten Kontrolltieren berechnet. Man bestimmt die $VD_{50}$ nach der Probitmethode. Die $VD_{50}$ ist diejenige vermizide Dosis, welche eine 50proz. Reduktion der Wurmzahl bewirkt.

In der nachfolgenden Tabelle werden die Resultate dargestellt, welche mit repräsentativen erfindungsgemässen Produkten erhalten worden sind. Angegeben werden die $VD_{50}$ in mg/kg p.o. und die $DL_{50}$ in mg/kg bei einmaliger oraler Verabreichung an Mäusen.

Tabelle

| Verbindung der Formel I | $VD_{50}$ in mg/kg p.p | $DL_{50}$ in mg/kg p.o. |
|---|---|---|
| 9-Acridanon-(2-thiazolidinyliden)-hydrazon | 26 | 1250–2500 |
| 10-Methyl-9-acridanon-(2-thiazolyl)hydrazon-hydrochlorid | 9 | >5000 |

Die erfindungsgemässen Produkte können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinkapseln, Lösungen, Emulsionen oder Suspensionen, verabreicht werden.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Öle, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen.

Die pharmazeutischen Präparate können daneben noch Konservierungsmittel, Lösungsmittler, Stabilisierungsmittel, Netzmittel, Emulgiermittel, Süssmittel, Färbemittel, Aromatisierungsmittel, Salze zur Veränderung des osmotischen Drukkes, Puffer, Überzugsmittel oder Antioxidantien enthalten. Sie können auch noch andere therapeutisch wertvolle Stoffe enthalten.

Wie eingangs erwähnt sind Arzneimittel, enthaltend ein Acridanon-Derivat der Formel I oder ein pharmazeutisch annehmbares Säureadditionssalz einer solchen Verbindung ebenfalls Gegenstand der vorliegenden Erfindung, weiterhin auch ein Verfahren zur Herstellung solcher Arzneimittel, welches dadurch gekennzeichnet ist, dass man eine oder mehrere erfindungsgemässe Stoffe und gegebenenfalls einen oder mehrere andere therapeutisch wertvolle Stoffe in eine galenische Darreichungsform bringt.

Wie eingangs erwähnt, können die erfindungsgemässen Produkte bei der Bekämpfung bzw. Verhütung von Krankheiten verwendet werden. Sie eignen sich insbesondere zur Bekämpfung bzw. Verhütung von Schistosomiasen. Die Dosierung kann innerhalb weiter Grenzen variieren und ist natürlich in jedem einzelnen Fall den individuellen Gegebenheiten anzupassen. Im allgemeinen dürfte eine einmalige orale Verabreichung von etwa 1 bis etwa 50 mg/kg Körpergewicht für die Behandlung von Schistosomiasen angemessen sein.

Die nachfolgenden Beispiele sollen die vorliegende Erfindung näher erläutern, ihren Umfang jedoch in keiner Weise beschränken. Alle Temperaturen sind in Celsiusgraden angegeben.

Beispiel 1

a) Man löst 1,0 g (4,7 mMol) 9-Chloracridin, 0,926 g (4,6 mMol) 2-Hydrazino-2-thiazolin-hydrobromid und 0,767 g (9,3 mMol) wasserfreies Natriumacetat in 100 ml trockenem Äthanol, erhitzt während 2 Stunden unter Rückfluss zum Sieden und dampft die rote Lösung dann im Vakuum ein. Man versetzt den Rückstand mit Wasser und saugt die kristalline Masse ab. Nach Umkristallisieren aus Äthanol erhält man 9-Acridanon-(2-thiazolidinyliden)hydrazon vom Schmelzpunkt 212–216° (Zersetzung).

In analoger Weise erhält man:

b) Aus 9-Chloracridin und N-Methyl-N-(2-thiazolin-2-yl)hydrazin-hydrochlorid das 9-Acridanon-methyl(2-thiazolin-2-yl)hydrazon vom Schmelzpunkt 242–243°.

Beispiel 2

Man löst 2,09 g (10 mMol) 9-Methoxyacridin in 50 ml trockenem Äthanol, versetzt mit einer Lösung von 1,98 g (10 mMol) 2-Hydrazino-2-thiazolin-hydrobromid in 50 ml trockenem Äthanol, erhitzt während 1 Stunde unter Rückfluss zum Sieden und entfernt das Lösungsmittel im Vakuum. Man versetzt den Rückstand mit wässrigem Ammoniak (1:5) und extrahiert mit Essigester. Nach Trocknen des Extraktes über Natriumsulfat und Eindampfen wird das erhaltene Material durch Zugabe von Pentan kristallisiert. Man erhält 9-Acridanon-(2-thiazolidinyliden)hydrazon vom Schmelzpunkt 214–215° (Zersetzung).

Beispiel 3

Man rührt eine Mischung aus 2,3 g 9-Chlor-1-nitro-acridin, 1,65 g N-Methyl-N-(2-thiazolyl)hydrazin-hydrochlorid und 100 ml Dimethylformamid während etwa 18 Stunden bei Raumtemperatur. Die dunkelrote Lösung wird eingedampft, worauf man den Rückstand mit Sodalösung alkalisch stellt und mit Methylenchlorid extrahiert. Der Auszug wird mit Wasser gewaschen, über Natriumsulfat getrocknet und eingedampft. Den Rückstand nimmt man in Äthanol auf, säuert mit äthanolischer Salzsäure an und fällt das Produkt durch Zugabe von Aether aus. Nach Abfiltrieren und Waschen mit Äther und Petroläther wird das hygroskopische Salz über Phosphorpentoxid getrocknet. Man erhält 1-Nitro-9-acridanon-methyl(2-thiazolyl)hydrazon-hydrochlorid vom Schmelzpunkt 185° (Zersetzung).

Beispiel 4

Man löst 5,0 g (19,3 mMol) 9-Chlor-1-nitroacridin, 3,83 g (19,3 mMol) 2-Hydrazino-2-thiazolin-hydrobromid und 3,17 g (38,6 mMol) wasserfreies Natriumacetat in 250 ml trockenem Methanol, erhitzt während 1 Stunde auf dem Dampfbad und lässt dann über Nacht stehen. Das ausgefallene Material wird abfiltriert und aus Methanol umkristallisiert. Man erhält 1-Nitro-9-acridanon-(2-thiazolidinyliden)-hydrazon das sich ab 152° zu zersetzen beginnt.

Beispiel 5

3,12 g 9-Acridanon-thiosemicarbazon und 2,33 g 1-Brom-2-butylamin-hydrobromid werden in 100 ml Äthanol während 15 Stunden unter Rückfluss zum Sieden erhitzt. Die noch heisse Lösung wird filtriert und eingedampft, worauf man den Rückstand mit Sodalösung alkalisch stellt und mit Methylenchlorid extrahiert. Der Auszug wird mit Wasser gewaschen, über Natriumsulfat getrocknet und eingedampft. Nach zweimaligem Umkristallisieren aus Aethanol erhält man 9-Acridanon-[2-(4-äthyl)thiazolidinyliden]hydrazon vom Schmelzpunkt 101–103°.

Beispiel 6

6,24 g 9-Acridanon-thiosemicarbazon, 3,5 g Natriumacetat und 5,2 ml Chloracetaldehyd (50proz. wässrige Lösung) in 200 ml Methanol werden während 6 Stunden unter Rückfluss zum Sieden erhitzt. Nach Abdampfen des Lösungsmittels versetzt man den Rückstand mit Wasser. Das ausgefallene Produkt wird abfiltriert und nacheinander mit Wasser, wenig Äthanol, Äther und Petroläther gewaschen. Man erhält 9-Acridanon-(2-thiazolyl)hydrazon vom Schmelzpunkt 208–210° (Zersetzung).

Beispiel 7

a) 3,3 g 10-Äthyl-9-acridanon werden während 1,5 Stunden in 50 ml Phosphoroxychlorid unter Rückfluss zum Sieden erhitzt, worauf die Lösung eingedampft wird. Man wäscht den Rückstand (10-Äthyl-9-chlor-acridinium-dichlorphosphat) mit Äther, versetzt mit 6,6 g Natriumacetat, 2,96 g

2-Hydrazino-2-thiazolin-hydrobromid und 100 ml Methanol und erhitzt das Gemisch unter Rückfluss zum Sieden. Nach 1 Stunde wird das auskristallisierte Material abfiltriert und nacheinander mit Wasser, Methanol und Äther gewaschen. Man erhält 10-Äthyl-9-acridanon-(2-thiazolidinyliden)-hydrazon vom Schmelzpunkt 187–188°.

In analoger Weise erhält man:

b) Aus 10-Methyl-9-acridanon und 2-Hydrazino-2-thiazolin-hydrobromid das 10-Methyl-9-acridanon-(2-thiazolidinyliden)hydrazon vom Schmelzpunkt 180–181°;

c) aus 10-(n-Butyl)-9-acridanon und 2-Hydrazino-2-thiazolin-hydrobromid das 10-(n-Butyl)-9-acridanon-(2-thiazolidinyliden)hydrazon vom Schmelzpunkt 170–172°;

d) aus 10-Allyl-9-acridanon und 2-Hydrazino-2-thiazolin-hydrobromid das 10-Allyl-9-acridanon-(2-thiazolidinyliden)-hydrazon vom Schmelzpunkt 219–221°;

e) aus 10-(2-Methoxyäthyl)-9-acridanon und 2-Hydrazino-2-thiazolin-hydrobromid das 10-(2-Methoxyäthyl)-9-acridanon-(2-thiazolidinyliden)hydrazon vom Schmelzpunkt 188–189°.

Beispiel 8

4,8 g 1-Nitro-9-acridanon werden in 200 ml Dimethylformamid gelöst und mit 0,5 g Natriumhydrid behandelt. Die tiefrote Lösung versetzt man mit 2,5 ml Methyljodid und rührt während 4 Stunden bei Raumtemperatur. Der erhaltene gelbe Niederschlag wird abfiltriert und nacheinander mit Dimethylformamid, Wasser, Äthanol und Äther gewaschen. Man erhält 10-Methyl-1-nitro-9-acridanon vom Schmelzpunkt 308–309°.

4 g 10-Methyl-1-nitro-9-acridanon werden in 100 ml Phosphoroxychlorid während 2 Stunden unter Rückfluss zum Sieden erhitzt, worauf man die trübe Lösung filtriert und eindampft. Man wäscht den Rückstand (1,9-Dichlor-10-methyl-acridinium-dichlorphosphat) mit Äther, versetzt mit 100 ml Methanol, 3,1 g 2-Hydrazino-2-thiazolin-hydrobromid und 4 g Natriumacetat und erhitzt unter Rückfluss zum Sieden. Nach 0,5 Stunden kühlt man ab und filtriert das rote Kristallisat ab. Zur Reinigung suspendiert man es in Methanol, säuert mit äthanolischer Salzsäure an und filtriert. Das Filtrat wird konzentriert und mit Diäthylamin basisch gestellt, worauf man das ausgefallene Produkt abfiltriert und nacheinander mit Äthanol, Wasser und wieder mit Äthanol wäscht. Nach Umkristallisieren aus n-Butanol erhält man 1-Chlor-10-methyl-9-acridanon-(2-thiazolidinyliden)hydrazon vom Schmelzpunkt 206–207°.

Beispiel 9

1,8 g 10-Methyl-9-acridanon werden in 50 ml Phosphoroxychlorid während 1 Stunde unter Rückfluss zum Sieden erhitzt, worauf man die Lösung im Vakuum eindampft. Der Rückstand wird in Äther aufgeschlämmt und abfiltriert. Das getrocknete grünliche Pulver (9-Chlor-10-methyl-acridiniumdichlorphosphat) löst man in 100 ml Methanol, versetzt mit 0,8 g Thiosemicarbazid und rührt während 1 Stunde bei Raumtemperatur. Das ausgefallene Produkt wird abfiltriert, mit Äthanol und Äther gewaschen und mit 50 ml konzentriertem Ammoniak während 10 Minuten zum Sieden erhitzt. Man filtriert heiss ab und wäscht nacheinander mit Wasser, Äthanol und Äther. Man erhält 10-Methyl-9-acridanon-thiosemicarbazon vom Schmelzpunkt 196–198° (Zersetzung).

1,9 g 10-Methyl-9-acridanon-thiosemicarbazon werden in 100 ml Methanol suspendiert und mit 1,73 ml Chloracetaldehyd (50proz. wässrige Lösung) während 6 Stunden bei 50° gerührt. Man konzentriert, filtriert das kristalline Produkt ab und wäscht nacheinander mit Äthanol, Wasser, Äthanol und Äther. Man erhält 10-Methyl-9-acridanon-(2-thiazolyl)hydrazon-hydrochlorid vom Schmelzpunkt 224–225° (Zersetzung).

Beispiel A

Herstellung von Tabletten nachstehender Zusammensetzung:

| | mg/Tablette |
|---|---|
| 10-Methyl-9-acridanon-(2-thiazolyl)-hydrazon | 100 |
| Milchzucker | 100 |
| Maisstärke | 85 |
| Povidon | 10 |
| Talk | 3 |
| Magnesiumstearat | 2 |
| Tablettengewicht | 300 |

Man vermischt den Wirkstoff mit dem Milchzucker und der Maisstärke, befeuchtet mit einer wässrigen Lösung von Povidon und granuliert. Das Granulat wird bei 40° getrocknet und gesiebt. Man vermischt mit dem Talk und dem Magnesiumstearat und verpresst zu Tabletten.

**Patentansprüche für die Vertragsstaaten BE, CH, DE, FR, GB, IT, LI, LU, NL, SE**

1. Acridanon-Derivate der allgemeinen Formel

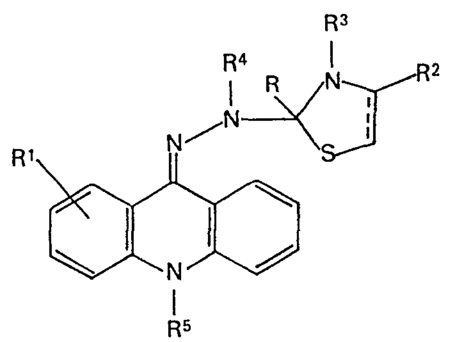

I

worin die punktierte Linie eine fakultative Bindung, $R^1$ Wasserstoff, Halogen oder Nitro, $R^2$ Wasserstoff oder niederes Alkyl, einer der Substituenten $R^3$ und $R^4$ Wasserstoff oder niederes Alkyl und der andere zusammen mit R eine zusätzliche Bindung und $R^5$ Wasserstoff, niederes Alkyl, niederes Alkenyl, niederes Alkinyl oder durch Halogen oder niederes Alkoxy substituiertes niederes Alkyl bedeuten, wobei die mit nieder bezeichneten Reste bis zu vier Kohlenstoffatome

enthalten und pharmazeutisch annehmbare Säureadditionssalze davon.

2. Verbindungen gemäss Anspruch 1, worin der Substituent $R^1$ sich in der 1-Stellung befindet.

3. Verbindungen gemäss Anspruch 1, worin $R^1$ Wasserstoff bedeutet.

4. Verbindungen gemäss einem der Ansprüche 1 bis 3, worin $R^2$ Wasserstoff bedeutet.

5. Verbindungen gemäss einem der Ansprüche 1 bis 4, worin einer der Substituenten $R^3$ und $R^4$ Wasserstoff und der andere zusammen mit R eine zusätzliche Bindung bedeuten.

6. Verbindungen gemäss einem der Ansprüche 1 bis 5, worin $R^5$ Wasserstoff oder niederes Alkyl bedeutet.

7. 10-Methyl-9-acridanon-(2-thiazolyl)hydrazon.

8. 9-Acridanon-(2-thiazolidinyliden)hydrazon, 9-Acridanon-(2-thiazolyl)hydrazon, 10-Methyl-9-acridanon-(2-thiazolidinyliden)hydrazon, 10-Äthyl-9-acridanon-(2-thiazolidinyliden)hydrazon, 9-Acridanon-methyl(2-thiazolinyl)hydrazon und 10-(2-Methoxyäthyl)-9-acridanon-(2-thiazolidinyliden)hydrazon.

9. Verbindungen gemäss einem der Ansprüche 1 bis 8 als pharmazeutische Wirkstoffe.

10. Verbindungen gemäss einem der Ansprüche 1 bis 8 als schistosomizide Stoffe.

11. 10-Methyl-9-acridanon-(2-thiazolyl)hydrazon als pharmazeutischer Wirkstoff.

12. 10-Methyl-9-acridanon-(2-thiazolyl)hydrazon als schistosomizider Stoff.

13. Verfahren zur Herstellung von Verbindungen gemäss einem der Ansprüche 1 bis 8, dadurch gekennzeichnet, dass man

a) eine Verbindung der allgemeinen Formel

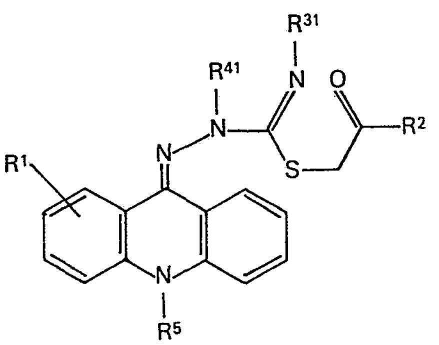

II

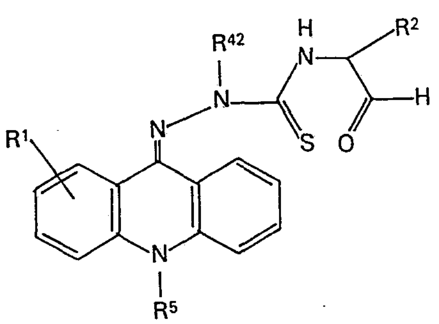

III

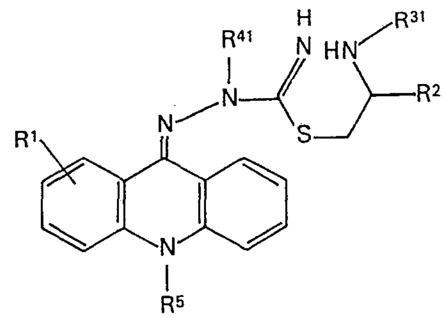

IV

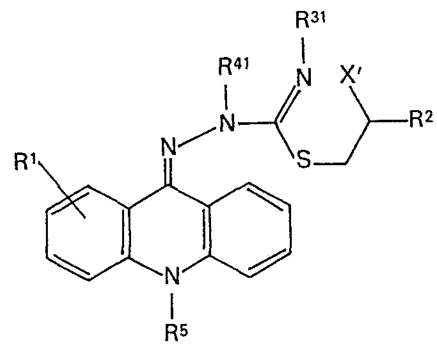

V

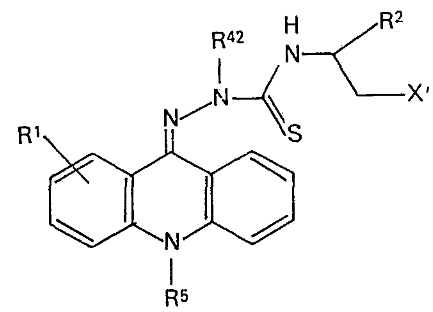

VI

oder

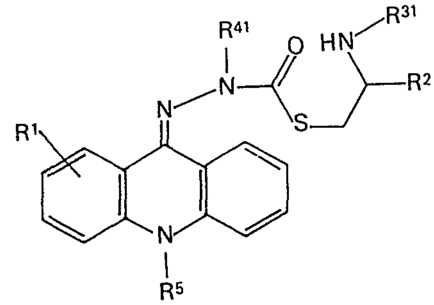

VII

worin einer der Substituenten $R^{31}$ und $R^{41}$ Wasserstoff oder niederes Alkyl und der andere Wasserstoff, $R^{42}$ Wasserstoff oder niederes Alkyl und X′ ein Halogenatom bedeuten, und $R^1$, $R^2$ und $R^5$ die in Anspruch 1 angegebene Bedeutung besitzen, cyclisiert, oder

b) eine Verbindung der allgemeinen Formel

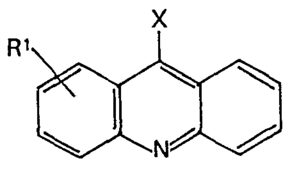

VIII

oder

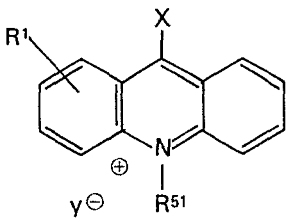

IX

worin $R^{51}$ niederes Alkyl, niederes Alkenyl, niederes Alkinyl oder durch Halogen oder niederes Alkoxy substituiertes niederes Alkyl, X eine Abgangsgruppe und $Y^{\ominus}$ ein Anion bedeuten, und $R^1$ die in Anspruch 1 angegebene Bedeutung besitzt,

mit einer Verbindung der allgemeinen Formel

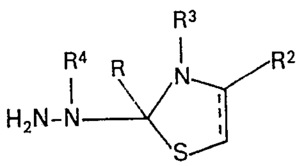

X

worin die punktierte Linie, R, $R^2$, $R^3$ und $R^4$ die in Anspruch 1 angegebene Bedeutung besitzen, umsetzt, oder

c) eine Verbindung der allgemeinen Formel

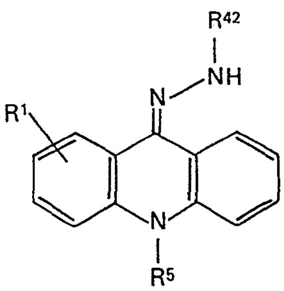

XI

worin $R^{42}$ Wasserstoff oder niederes Alkyl bedeutet, und $R^1$ und $R^5$ die in Anspruch 1 angegebene Bedeutung besitzen,

mit einer Verbindung der allgemeinen Formel

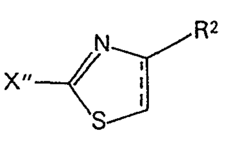

XII

worin X″ eine Abgangsgruppe bedeutet und $R^2$ und die punktierte Linie die in Anspruch 1 angegebene Bedeutung besitzen, umsetzt, und

d) erwünschtenfalls eine erhaltene Verbindung der Formel I in ein pharmazeutisch annehmbares Säureadditionssalz überführt.

14. Verfahren gemäss Anspruch 13, dadurch gekennzeichnet, dass man 10-Methyl-9-acridanon-(2-thiazolyl)-hydrazon herstellt.

15. Arzneimittel, enthaltend eine Verbindung gemäss einem der Ansprüche 1 bis 8.

16. Schistosomizide Mittel, enthaltend eine Verbindung gemäss einem der Ansprüche 1 bis 8.

17. Arzneimittel, enthaltend 10-Methyl-9-acridanon-(2-thiazolyl)hydrazon.

18. Schistomizide Mittel, enthaltend 10-Methyl-9-acridanon-(2-thiazolyl)hydrazon.

19. Verbindungen der in Anspruch 13 definierten allgemeinen Formeln II, III, IV, V, VI und VII.

**Patentansprüche für den Vertragsstaat AT**

1. Verfahren zur Herstellung von Acridanon-Derivaten der allgemeinen Formel

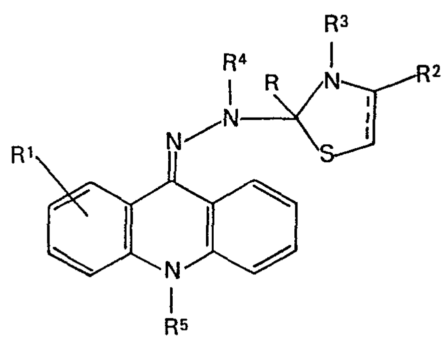

I

worin die punktierte Linie eine fakultative Bindung, $R^1$ Wasserstoff, Halogen oder Nitro, $R^2$ Wasserstoff oder niederes Alkyl, einer der Substituenten $R^3$ und $R^4$ Wasserstoff oder niederes Alkyl und der andere zusammen mit R eine zusätzliche Bindung und $R^5$ Wasserstoff, niederes Alkyl, niederes Alkenyl, niederes Alkinyl oder durch Halogen oder niederes Alkoxy substituiertes niederes Alkyl bedeuten, wobei die mit nieder bezeichneten Reste bis zu vier Kohlenstoffatome enthalten und pharmazeutisch annehmbare Säureadditionssalze davon, dadurch gekennzeichnet, dass man

a) eine Verbindung der allgemeinen Formel

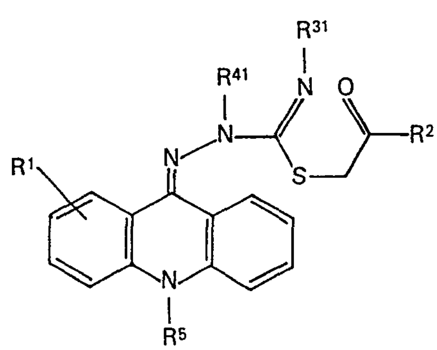

II

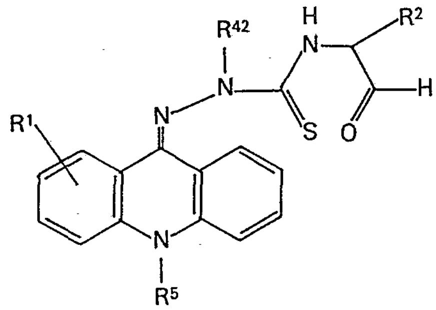

III

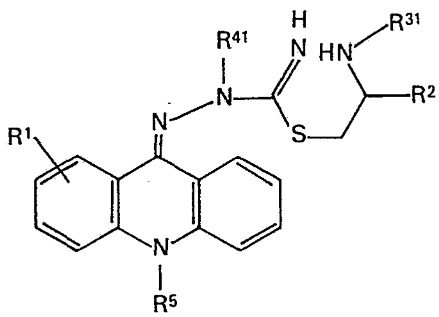

IV

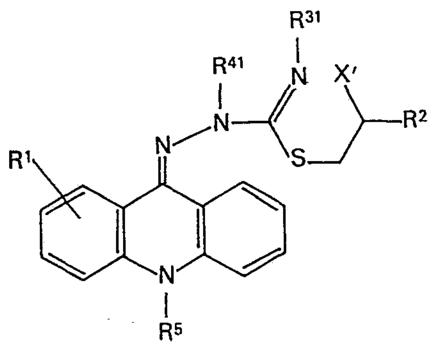

V

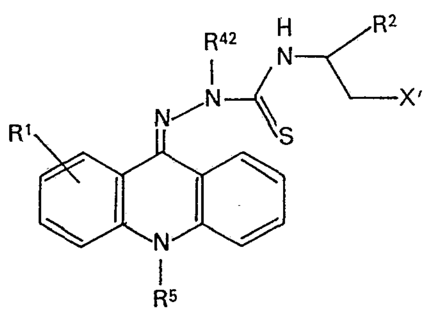

VI

oder

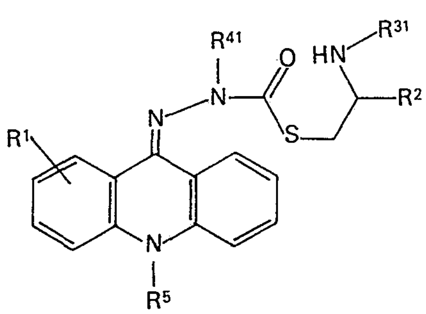

VII

worin einer der Substituenten $R^{31}$ und $R^{41}$ Wasserstoff oder niederes Alkyl und der andere Wasserstoff, $R^{42}$ Wasserstoff oder niederes Alkyl und X′ ein Halogenatom bedeuten, und $R^1$, $R^2$ und $R^5$ die in Anspruch 1 angegebene Bedeutung besitzen, cyclisiert, oder

b) eine Verbindung der allgemeinen Formel

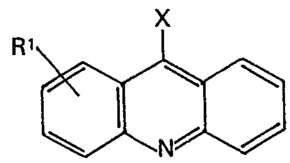

VIII

oder

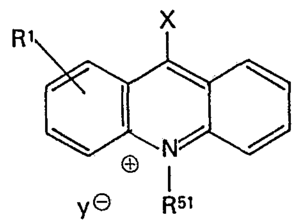

IX

worin $R^{51}$ niederes Alkyl, niederes Alkenyl, niederes Alkinyl oder durch Halogen oder niederes Alkoxy substituiertes niederes Alkyl, X eine Abgangsgruppe und $Y^{\ominus}$ ein Anion bedeuten, und $R^1$ die in Anspruch 1 angegebene Bedeutung besitzt,

mit einer Verbindung der allgemeinen Formel

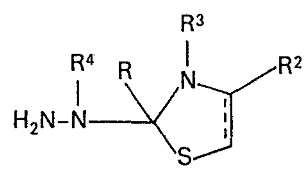

X

worin die punktierte Linie, R, $R^2$, $R^3$ und $R^4$ die in Anspruch 1 angegebene Bedeutung besitzen, umsetzt, oder

c) eine Verbindung der allgemeinen Formel

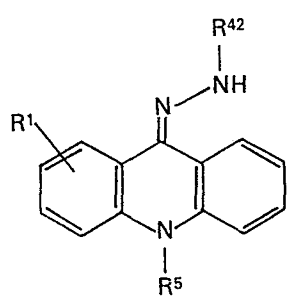

XI

worin $R^{42}$ Wasserstoff oder niederes Alkyl bedeutet, und $R^1$ und $R^5$ die in Anspruch 1 angegebene Bedeutung besitzen, mit einer Verbindung der allgemeinen Formel

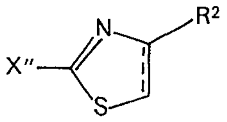

XII

worin X″ eine Abgangsgruppe bedeutet und $R^2$ und die punktierte Linie die in Anspruch 1 angegebene Bedeutung besitzen, umsetzt, und

d) erwünschtenfalls eine erhaltene Verbindung der Formel I in ein pharmazeutisch annehmbares Säureadditionssalz überführt.

2. Verfahren gemäss Anspruch 1, dadurch gekennzeichnet, dass man Verbindungen der Formel I herstellt, worin der Substituent $R^1$ sich in der 1-Stellung befindet.

3. Verfahren gemäss Anspruch 1, dadurch gekennzeichnet, dass man Verbindungen der Formel I herstellt, worin $R^1$ Wasserstoff bedeutet.

4. Verfahren gemäss einem der Ansprüche 1 bis 3, dadurch gekennzeichnet, dass man Verbindungen der Formel I herstellt, worin $R^2$ Wasserstoff bedeutet.

5. Verfahren gemäss einem der Ansprüche 1 bis 4, dadurch gekennzeichnet, dass man Verbindungen der Formel I herstellt, worin einer der Substituenten $R^3$ und $R^4$ Wasserstoff und der andere zusammen mit R eine zusätzliche Bindung bedeuten.

6. Verfahren gemäss einem der Ansprüche 1 bis 5, dadurch gekennzeichnet, dass man Verbindungen der Formel I herstellt, worin $R^5$ Wasserstoff oder niederes Alkyl bedeutet.

7. Verfahren nach Anspruch 1, dadurch gekennzeichnet, dass man 10-Methyl-9-acridanon-(2-thiazolyl)hydrazon herstellt.

8. Verfahren nach Anspruch 1, dadurch gekennzeichnet, dass man 9-Acridanon-(2-thiazolidinyliden)hydrazon, 9-Acridanon-(2-thiazolyl)hydrazon, 10-Methyl-9-acridanon-(2-thiazolyl)hydrazon, 10-Methyl-9-acridanon-(2-thiazolidinyliden)hydrazon, 10-Äthyl-9-acridanon-(2-thiazolidinyliden)hydrazon, 9-Acridanon-methyl(2-thiazolinyl)hydrazon und 10-(2-Methoxyäthyl)-9-acridanon-(2-thiazolidinyliden)hydrazon herstellt.

**Claims for the Contracting States BE, CH, DE, FR, GB, IT, LI, LU, NL, SE**

1. Acridanone derivatives of the general formula

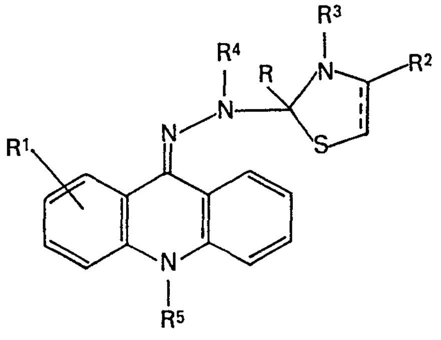

I

wherein the dotted line signifies an optional bond, $R^1$ signifies hydrogen, halogen or nitro, $R^2$ signifies hydrogen or lower alkyl, one of the substituents $R^3$ and $R^4$ signifies hydrogen or lower alkyl and the other together with R signifies an additional bond and $R^5$ signifies hydrogen, lower alkyl, lower alkenyl, lower alkynyl or lower alkyl substituted by halogen or lower alkoxy, whereby the residues denoted by lower contain up to four carbon atoms,

and pharmaceutically acceptable acid addition salts thereof.

2. Compounds in accordance with claim 1, wherein the substituent $R^1$ is situated in the 1-position.

3. Compounds in accordance with claim 1, wherein $R^1$ signifies hydrogen.

4. Compounds in accordance with any one of claims 1 to 3, wherein $R^2$ signifies hydrogen.

5. Compounds in accordance with any one of claims 1 to 4, wherein one of the substituents $R^3$ and $R^4$ signifies hydrogen and the other together with R signifies an additional bond.

6. Compounds in accordance with any one of claims 1 to 5, wherein $R^5$ signifies hydrogen or lower alkyl.

7. 10-Methyl-9-acridanone (2-thiazolyl)hydrazone.

8. 9-Acridanone (2-thiazolidinylidene)hydrazone, 9-acridanone (2-thiazolyl)hydrazone, 10-methyl-9-acridanone (2-thiazolidinylidene)hydrazone, 10-ethyl-9-acridanone (2-thiazolidinylidene)hydrazone, 9-acridanone methyl(2-thiazolinyl)hydrazone and 10-(2-methoxyethyl)-9-acridanone (2-thiazolidinylidene)hydrazone.

9. Compounds in accordance with any one of claims 1 to 8 as pharmaceutically active substances.

10. Compounds in accordance with any one of claims 1 to 8 as schistosomicidal substances.

11. 10-Methyl-9-acridanone (2-thiazolyl)hydrazone as a pharmaceutically active substance.

12. 10-Methyl-9-acridanone (2-thiazolyl)hydrazone as a schistosomicidal substance.

13. A process for the manufacture of compounds in accordance with any one of claims 1 to 8, characterized by

a) cyclizing a compound of the general formula

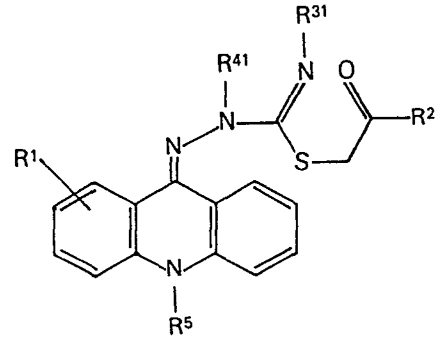

II

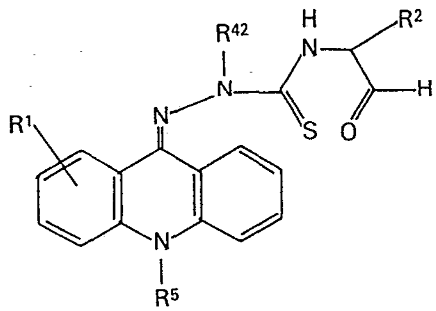

III

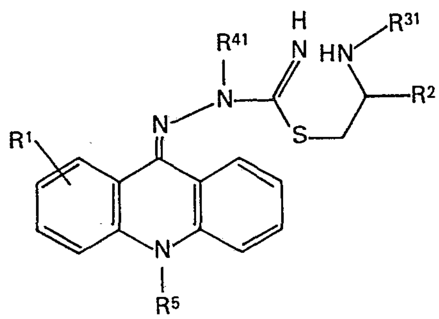

IV

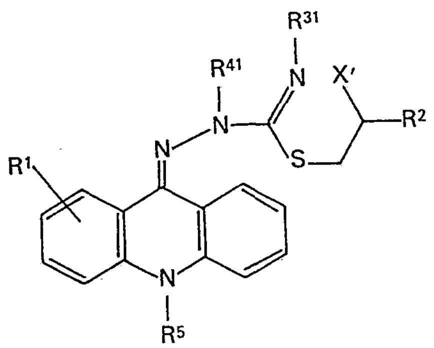

V

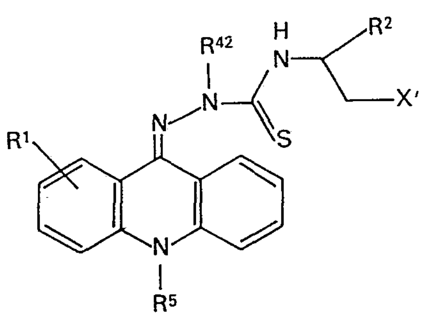

VI

or

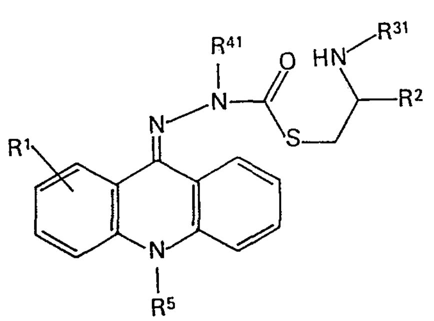

VII

wherein one of the substituents $R^{31}$ and $R^{41}$ signifies hydrogen or lower alkyl and the other signifies hydrogen, $R^{42}$ signifies hydrogen or lower alkyl and X′ signifies a halogen atom, and $R^1$, $R^2$ and $R^5$ have the significance given in claim 1,

b) reacting a compound of the general formula

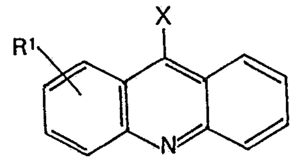

VIII

or

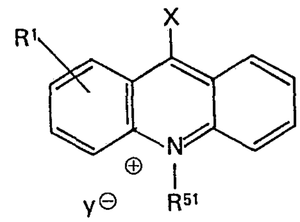

IX

wherein $R^{51}$ signifies lower alkyl, lower alkenyl, lower alkynyl or lower alkyl substituted by halogen or lower alkoxy, X signifies a leaving group and $Y^{\ominus}$ signifies an anion, and $R^1$ has the significance given in claim 1,

with a compound of the general formula

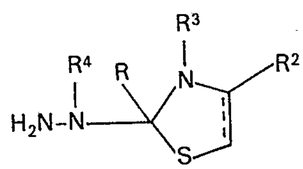

X

wherein the dotted line, R, $R^2$, $R^3$ and $R^4$ have the significance given in claim 1, or

c) reacting a compound of the general formula

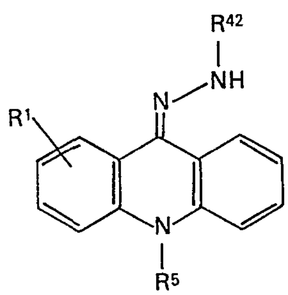

XI

wherein $R^{42}$ signifies hydrogen or lower alkyl, and $R^1$ and $R^5$ have the significance given in claim 1, with a compound of the general formula

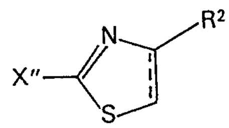

XII

wherein X″ signifies a leaving group and $R^2$ and the dotted line have the significance given in claim 1, and

d) if desired, converting a compound of formula I obtained into a pharmaceutically acceptable acid addition salt.

14. A process in accordance with claim 13, characterized in that 10-methyl-9-acridanone (2-thiazolyl)hydrazone is manufactured.

15. A medicament containing a compound in accordance with any one of claims 1 to 8.

16. A schistosomicidal medicament containing a compound in accordance with any one of claims 1 to 8.

17. A medicament containing 10-methyl-9-acridanone (2-thiazolyl)hydrazone.

18. A schistosomidical medicament containing 10-methyl-9-acridanone (2-thiazolyl)hydrazone.

19. Compounds of general formulae II, III, IV, V, VI and VII defined in claim 13.

**Claims for the Contracting State AT**

1. A process for the manufacture of acridanone derivatives of the general formula

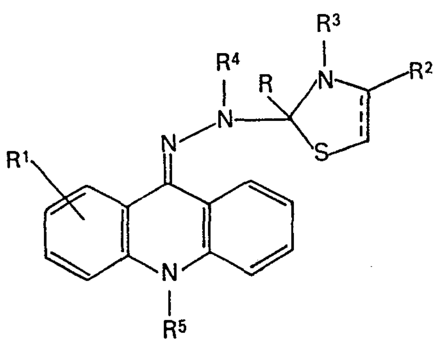

I

wherein the dotted line signifies an optional bond, $R^1$ signifies hydrogen, halogen or nitro, $R^2$ signifies hydrogen or lower alkyl, one of the substituents $R^3$ and $R^4$ signifies hydrogen or lower alkyl and the other together with R signifies an additional bond and $R^5$ signifies hydrogen, lower alkyl, lower alkenyl, lower alkynyl or lower alkyl substituted by halogen or lower alkoxy, whereby the residues denoted by lower contain up to four carbon atoms.

and pharmaceutically acceptable acid addition salts thereof, characterized by

a) cyclizing a compound of the general formula

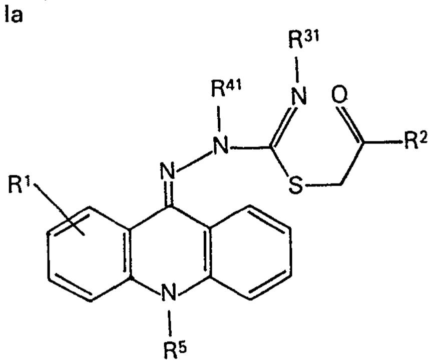

II

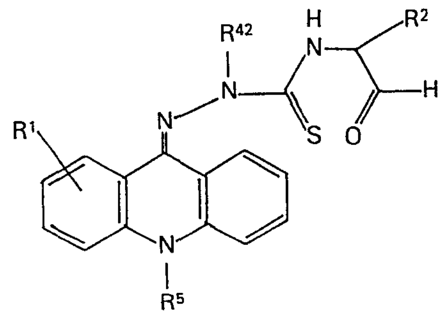

III

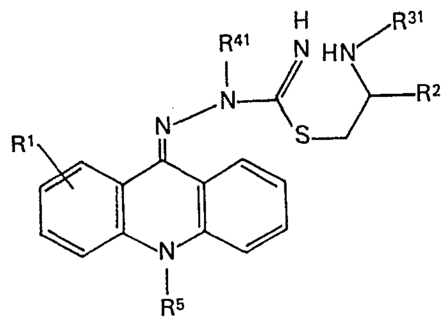

IV

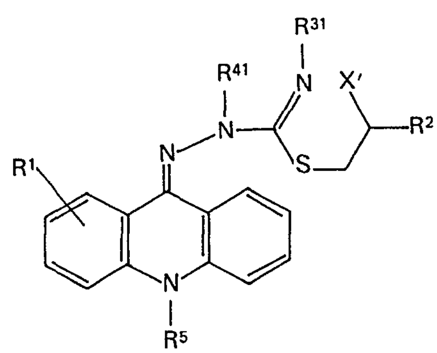

V

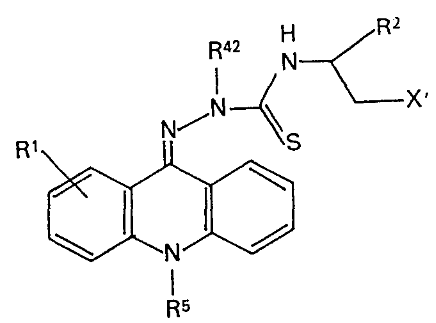

VI

or

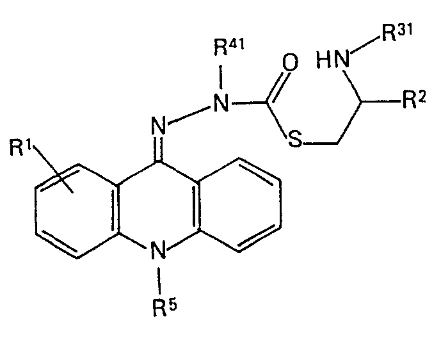

VII

wherein one of the substituents $R^{31}$ and $R^{41}$ signifies hydrogen or lower alkyl and the other signifies hydrogen, $R^{42}$ signifies hydrogen or lower alkyl and X′ signifies a halogen atom, and $R^1$, $R^2$ and $R^5$ have the significance given in claim 1, or

b) reacting a compound of the general formula

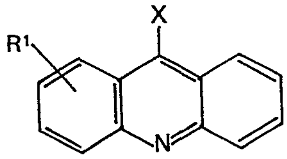

VIII

or

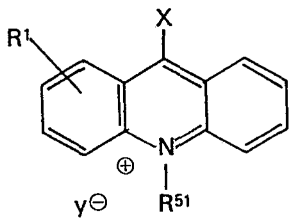

IX

wherein $R^{51}$ signifies lower alkyl, lower alkenyl, lower alkynyl or lower alkyl substituted by halogen or lower alkoxy, X signifies a leaving group and $Y^{\ominus}$ signifies an anion, and $R^1$ has the significance given in claim 1,

with a compound of the general formula

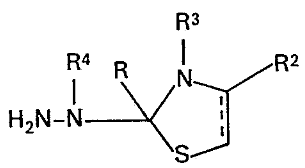

X

wherein the dotted line, R, $R^2$, $R^3$ and $R^4$ have the significance given in claim 2, or

c) reacting a compound of the general formula

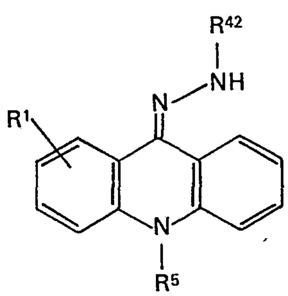

XI

wherein $R^{42}$ signifies hydrogen or lower alkyl, and $R^1$ and $R^5$ have the significance given in claim 1, with a compound of the general formula

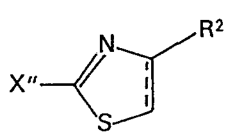

XII

wherein X″ signifies a leaving group and $R^2$ and the dotted line have the significance given in claim 1, and

d) if desired, converting a compound of formula I obtained into a pharmaceutically acceptable acid addition salt.

2. A process in accordance with claim 1, characterized in that compounds of formula I in which the substituent $R^1$ is situated in the 1-position are manufactured.

3. A process in accordance with claim 1, characterized in that compounds of formula I in which $R^1$ signifies hydrogen are manufactured.

4. A process in accordance with any one of claims 1 to 3, characterized in that compounds of formula I in which $R^2$ signifies hydrogen are manufactured.

5. A process in accordance with any one of claims 1 to 4, characterized in that compounds of formula I in which one of the substituents $R^3$ and $R^4$ signifies hydrogen and the other together with R signifies an additional bond are manufactured.

6. A process in accordance with any one of claims 1 to 5, characterized in that compounds of formula I in which $R^5$ signifies hydrogen or lower alkyl are manufactured.

7. A process according to claim 1, characterized in that 10-methyl-9-acridanone (2-thiazolyl)hydrazone is manufactured.

8. A process according to claim 1, characterized in that 9-acridanone (2-thiazolidinylidene)hydrazone, 9-acridanone (2-thiazolyl)hydrazone, 10-methyl-9-acridanone (2-thiazolidinylidene)hydrazone, 10-ethyl-9-acridanone (2-thiazolidinylidene)hydrazone, 9-acridanone methyl(2-thiazolinyl)hydrazone and 10-(2-methoxyethyl-9-acridanone (2-thiazolidinylidene)hydrazone are manufactured.

**Revendications pour les Etats contractants pour BE, CH, DE, FR, GB, IT, LI, LU, NL, SE**

1. Dérivés de l'acridanone, de formule générale

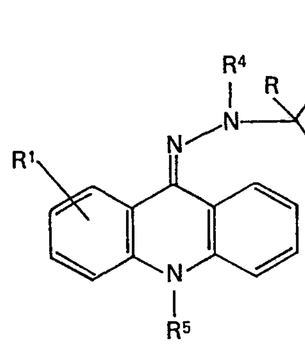

I

dans laquelle le trait en pointillé représente une liaison facultative, $R^1$ représente l'hydrogène, un halogène ou un groupe nitro, $R^2$ resprésente

l'hydrogène ou un groupe alkyle inférieur, l'un des symboles $R^3$ et $R^4$ représente l'hydrogène ou un groupe alkyle inférieur et l'autre forme avec R une liaison supplémentaire et $R^5$ représente l'hydrogène, un groupe alkyle inférieur, alcényle inférieur, alcynyle inférieur ou un groupe alkyle inférieur substitué par des halogènes ou un groupe alcoxy inférieur, les groupes qualifiés d'«inférieurs» contenant jusqu'à 4 atomes de carbone, et leurs sels acceptables pour l'usage pharmaceutique, formés par addition avec des acides.

2. Composés selon la revendication 1, dans lesquels le substituant $R^1$ se trouve en position 1.

3. Composés selon la revendication 1, dans lesquels $R^1$ représente l'hydrogène.

4. Composés selon l'une des revendications 1 à 3, dans lesquels $R^2$ représente l'hydrogène.

5. Composés selon l'une des revendications 1 à 4, dans lesquels l'un des symboles $R^3$ et $R^4$ représente l'hydrogène et l'autre forme avec R une liaison supplémentaire.

6. Composés selon l'une des revendications 1 à 5, dans lesquels $R^5$ représente l'hydrogène ou un groupe alkyle inférieur.

7. La 10-méthyl-9-acridanone-(2-thiazolyl)-hydrazone.

8. La 9-acridanone-(2-thiazolidinylidène)-hydrazone, la 9-acridanone-(2-thiazolyl)-hydrazone, la 10-méthyl-9-acridanone-(2-thiazolidinylidène)-hydrazone, la 10-éthyl 9-acridanone-(2-thiazolidinylidène)-hydrazone, la 9-acridanone-méthyl-(2-thiazolinyl)-hydrazone et la 10-(2-méthoxyéthyl)-9-acridanone-(2-thiazolidinylidène)-hydrazone.

9. Composés selon l'une des revendications 1 à 8, en tant que substances actives pharmaceutiques.

10. Composés selon l'une des revendications 1 à 8, en tant que substances schistosomicides.

11. La 10-méthyl-9-acridanone-(2-thiazolyl)-hydrazone en tant que substance active pharmaceutique.

12. La 10-méthyl-9-acridanone-(2-thiazolyl)-hydrazone en tant que substance schistosomicide.

13. Procédé de préparation des composés selon l'une des revendications 1 à 8, caractérisé en ce que

a) on cyclise un composé de formule générale

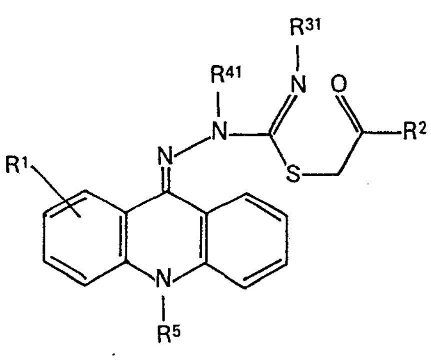

II

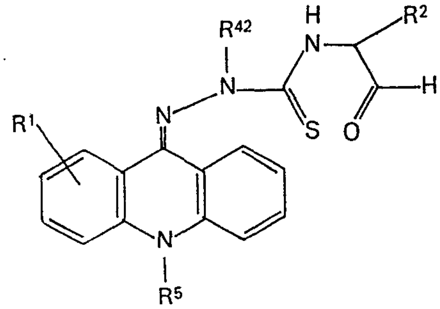

III

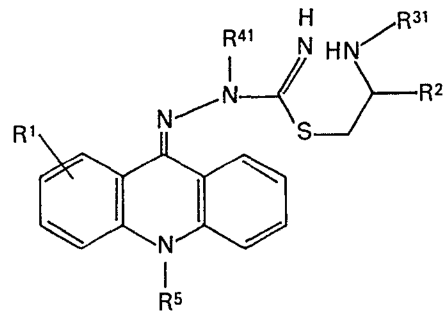

IV

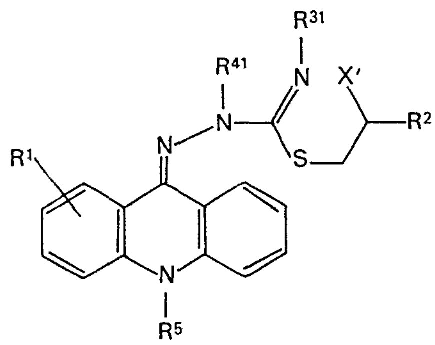

V

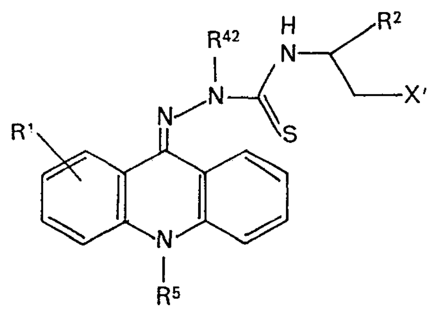

VI

ou

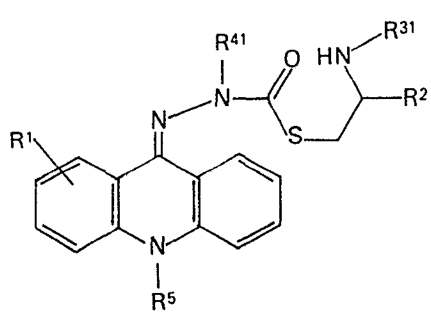

VII

dans lesquelles l'un des symboles $R^{31}$ et $R^{41}$ représente l'hydrogène ou un groupe alkyle inférieur et l'autre représente l'hydrogène, $R^{42}$ représente l'hydrogène ou un alkyle inférieur et X' un atome d'halogène, et $R^1$, $R^2$ et $R^5$ ont les significations indiquées dans la revendication 1, ou bien

b) on fait réagir un composé de formule générale

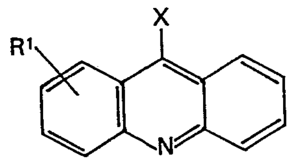

VIII

ou

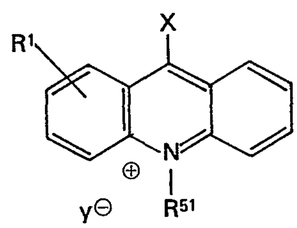

IX

dans laquelle $R^{51}$ représente un groupe alkyle inférieur, alcényle inférieur, alcynyle inférieur ou un groupe alkyle inférieur substitué par des halogènes ou un groupe alcoxy inférieur, X représente un groupe éliminable et $Y^{\ominus}$ représente un anion, $R^1$ ayant les significations indiquées dans la revendication 1,

avec un composé de formule générale

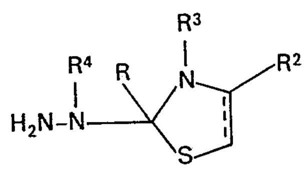

X

dans laquelle le trait en pointillé, R, $R^2$, $R^3$ et $R^4$ ont les significations indiquées dans la revendication 1, ou bien

c) on fait réagir un composé de formule générale

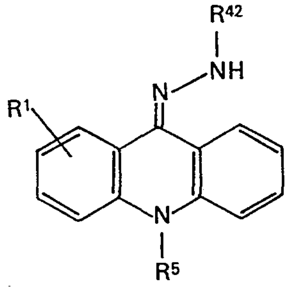

XI

dans laquelle $R^{42}$ représente l'hydrogène ou un groupe alkyle inférieur, et $R^1$ et $R^5$ ont les significations indiquées dans la revendication 1,

avec un composé de formule générale

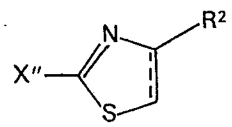

XII

dans laquelle X″ représente un groupe éliminable et $R^2$ et le trait en pointillé ont les significations indiquées dans la revendication 1, et

d) si on le désire, on convertit un composé obtenu, répondant à la formule I, en un sel acceptable pour l'usage pharmaceutique, par addition avec un acide.

14. Procédé selon la revendication 13, caractérisé en ce que l'on prépare la 10-méthyl-9-acridanone-(2-thiazolyl)-hydrazone.

15. Médicament contenant un composé selon l'une des revendications 1 à 8.

16. Agent schistosomicide contenant un composé selon l'une des revendications 1à 8.

17. Médicament contenant de la 10-méthyl-9-acridanone-(2-thiazolyl)-hydrazone.

18. Agent schistosomicide contenant de la 10-méthyl-9-acridanone-(2-thiazolyl)-hydrazone.

19. Composés répondant aux formules générales II, III, IV, V, VI et VII définies dans la revendication 13.

**Revendications pour l'Etat contractant: AT**

1. Procédé de préparation de dérivés de l'acridanone répondant à la formule générale

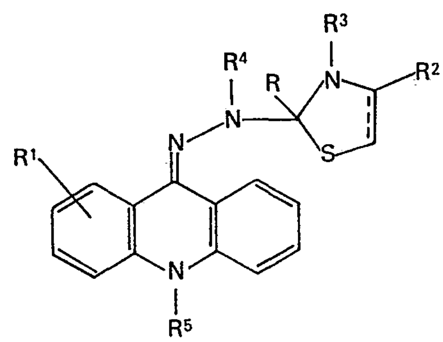

I

dans laquelle le trait en pointillé représente une liaison facultative, $R^1$ représente l'hydrogène, un halogène ou un groupe nitro, $R^2$ représente l'hydrogène ou un groupe alkyle inférieur, l'un des symboles $R^3$ et $R^4$ représente l'hydrogène ou un groupe alkyle inférieur et l'autre forme avec R une liaison supplémentaire, et $R^5$ représente l'hydrogène, un groupe alkyle inférieur, alcényle inférieur, alcynyle inférieur ou un groupe alkyle inférieur substitué par des halogènes ou par un groupe alcoxy inférieur, les groupes qualifiés d'«inférieurs» contenant jusqu'à 4 atomes de carbone, et de leurs sels acceptables pour l'usage pharmaceutique formés par addition avec un acide, caractérisé en ce que

a) on cyclise un composé de formule générale

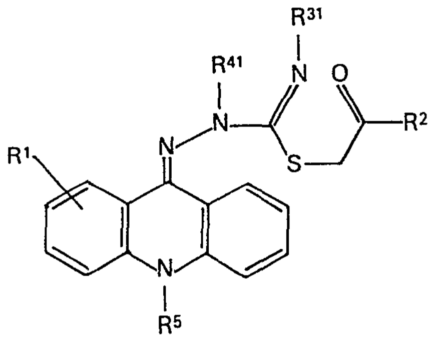

II

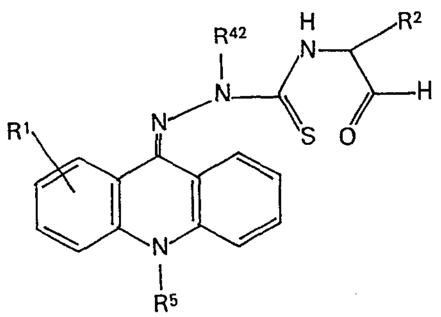

III

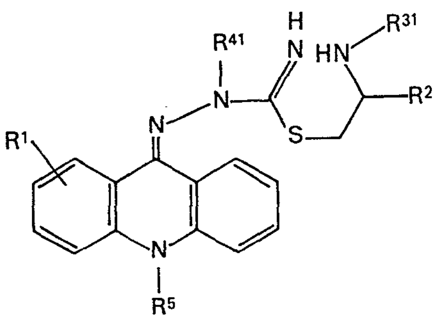

IV

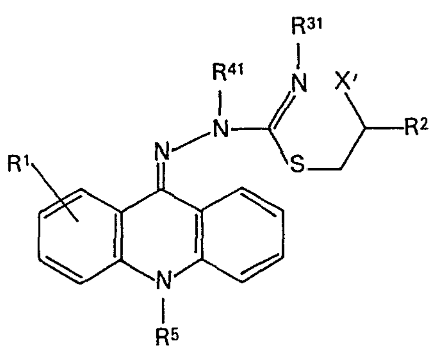

V

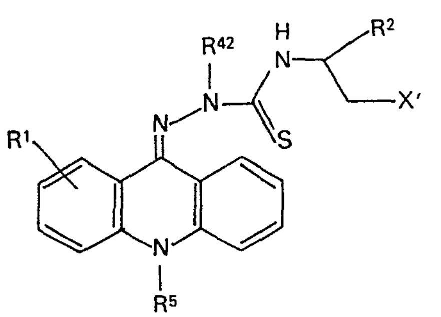

VI

ou

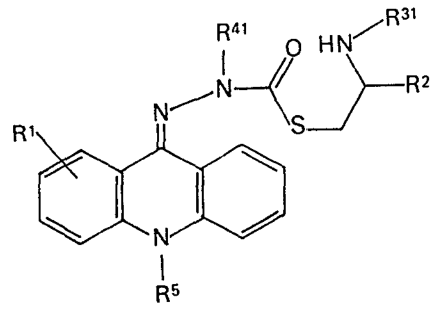

VII

dans lesquelles l'un des symboles $R^{31}$ et $R^{41}$ représente l'hydrogène ou un groupe alkyle inférieur et l'autre représente l'hydrogène, $R_{42}$ représente l'hydrogène ou un groupe alkyle inférieur et X' un atome d'halogène, et $R^1$, $R^2$ et $R^5$ ont les significations indiquées dans la revendication 1, ou bien

b) on fait réagir un composé de formule générale

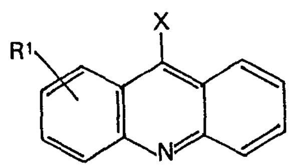

VIII

ou

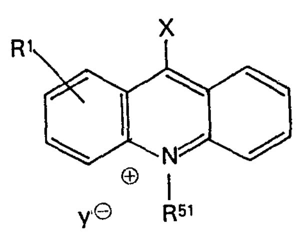

IX

dans laquelle $R^{51}$ représente un groupe alkyle inférieur, alcényle inférieur, alcynyle inférieur ou un groupe alkyle inférieur substitué par des halogènes ou un groupe alcoxy inférieur, X représente un groupe éliminable et $Y^{\ominus}$ représente un anion, $R^1$ ayant les significations indiquées dans la revendication 1, avec un composé de formule générale

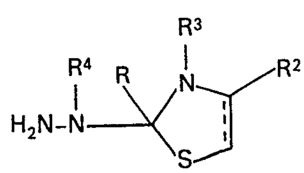

X

dans laquelle le trait en pointillé, R, $R^2$, $R^3$ et $R^4$ ont les significations indiquées dans la revendication 1, ou bien

c) on fait réagir un composé de formule générale

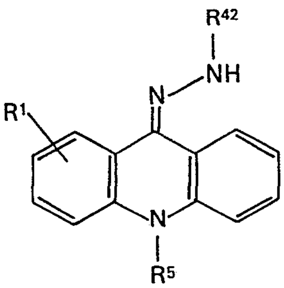

XI

dans laquelle $R^{42}$ représente l'hydrogène ou un groupe alkyle inférieur, et $R^1$ et $R^5$ ont les significations indiquées dans la revendication 1,

avec un composé de formule générale

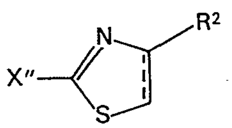

XII

dans laquelle X″ représente un groupe éliminable et $R^2$ et le trait en pointillé ont les significations indiquées dans la revendication 1, et

d) si on le désire, on convertit un composé obtenu, répondant à la formule générale I, en un sel acceptable pour l'usage pharmaceutique formé par addition avec un acide.

2. Procédé selon la revendication 1, caractérisé en ce que l'on prépare des composés de formule I dans lesquels le substituant $R^1$ se trouve en position 1.

3. Procédé selon la revendication 1, caractérisé en ce que l'on prépare des composés de formule I dans laquelle $R^1$ représente l'hydrogène.

4. Procédé selon l'une des revendications 1 à 3, caractérisé en ce que l'on prépare des composés de formule I dans laquelle $R^2$ représente l'hydrogène.

5. Procédé selon l'une des revendications 1 à 4, caractérisé en ce que l'on prépare des composés de formule I dans laquelle l'un des symboles $R^3$ et $R^4$ représente l'hydrogène et l'autre forme avec R une liaison supplémentaire.

6. Procédé selon l'une des revendications 1 à 5, caractérisé en ce que l'on prépare des composés de formule I dans laquelle $R^5$ représente l'hydrogène ou un groupe alkyle inférieur.

7. Procédé selon la revendication 1, caractérisé en ce que l'on prépare la 10-méthyl-9-acridanone-(2-thiazolyl)-hydrazone.

8. Procédé selon la revendication 1, caractérisé en ce que l'on prépare la 9-acridanone-(2-thiazolidinylidène)-hydrazone, la 9-acridanone-(2-thiazolyl)-hydrazone, la 10-méthyl-9-acridanone-(2-thiazolidinylidène)-hydrazone, la 10-éthyl-9-acridanone-(2-thiazolidinylidène)-hydrazone, la 9-acridanone-méthyl-(2-thiazolinyl)-hydrazone et la 10-(2-méthoxyéthyl)-9-acridanone-(2-thiazolidinylidène)-hydrazone.